(19) Europäisches Patentamt
European Patent Office
Office européen des brevets

(11) **EP 2 117 456 B1**

(12) **EUROPEAN PATENT SPECIFICATION**

(45) Date of publication and mention
of the grant of the patent:
**06.01.2016 Bulletin 2016/01**

(51) Int Cl.:
***A61B 18/18*** (2006.01)     ***A61B 5/05*** (2006.01)

(21) Application number: **08718623.5**

(86) International application number:
**PCT/GB2008/000762**

(22) Date of filing: **06.03.2008**

(87) International publication number:
**WO 2008/110756 (18.09.2008 Gazette 2008/38)**

(54) **TISSUE CLASSIFYING APPARATUS**

GEWEBEKLASSIFIZIERUNGSGERÄT

APPAREIL DE CLASSEMENT DE TISSUS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **09.03.2007 GB 0704650**

(43) Date of publication of application:
**18.11.2009 Bulletin 2009/47**

(73) Proprietor: **Medical Device Innovations Limited Cheshire WA4 4FS (GB)**

(72) Inventors:
• **HANCOCK, Christopher Paul Avon BS5 8QZ (GB)**
• **BISHOP, John Avon BS6 7LF (GB)**
• **BOOTON, Martin Wynford Somerset BA5 3PZ (GB)**

(74) Representative: **Johnson, Richard Alan et al Mewburn Ellis LLP City Tower 40 Basinghall Street London EC2V 5DE (GB)**

(56) References cited:
**WO-A-2004/047659     WO-A-2005/115235 US-A- 5 829 437**

**Description**

FIELD OF THE INVENTION

The invention relates to the treatment of biological tissue using microwave radiation. In particular, the invention relates to a tissue treatment system capable of measuring tissue properties using microwave radiation delivered from an antenna probe.

BACKGROUND TO THE INVENTION

An electrosurgical system that is arranged to controllably ablate biological tissue (e.g. a tumour) and/or measure information concerning the tumour and surrounding healthy tissue is known. Such a system may use two channels: a first channel to perform controlled tissue ablation, and a second channel to perform sensitive tissue state (dielectric) measurements. The general principles relating to the operation of such a system are disclosed in WO2004/047659 and WO2005/115235.

Fig. 1 shows a schematic diagram of the apparatus disclosed in WO2005/115235. The apparatus has a stable phase locked source of microwave radiation 1 connected to a probe 5 configured to direct the microwave radiation into tissue 6 to be measured or ablated. The probe 5 is adapted for insertion into the tissue, so that the tissue being measured is at or surrounding the distal end 5a of the probe 5.

On the path between the source 1 and the probe 5 there is an amplifier 2, a circulator 40 for isolating the probe 5 (which may have an output circuit comprising microwave connectors, a DC isolation barrier, waveguide or semi-rigid cable, a flexible cable assembly) from the amplifier 2 to prevent reflected power from damaging the amplifier 2, a triple stub impedance tuner 50 and a cable assembly 4. The impedance of tuner 50 is varied by movement of three tuning elements in and out of the tuning cavity. The tuning elements are moved by an actuator 1130, which is controlled by signals $A_1$, $A_2$, $A_3$ from a controller 101.

When the apparatus is used to direct microwave radiation through the probe 5 and into tissue 6 at the end of the probe 5, the tissue 6 will reflect a portion of the microwave radiation back through the probe 5 towards the source 1. A directional coupler 200 diverts a portion of this reflected signal 210 to an input B of a detector 100. A further directional coupler 250 couples a forward reference signal 255 to an input A of the detector 100.

The detector 100 detects the magnitude and phase of both the reflected signal 210 and the reference signal 255 and this information is used to enable the complex impedance of the tissue to be determined. The phase and magnitude information obtained from the detector may also be converted into other useful formats, for example, polar plots, or separate plots of phase variations with frequency and magnitude variations with frequency, i.e. so long as the phase and magnitude information can be extracted, the information may be presented in any format that provides useful information concerning the tissue type or state.

This information is then output to a tissue classifier 150 which classifies the tissue 6 as a particular tissue type (e.g. muscle, fat, cancerous tumour) and outputs the result to a display 160, which displays the tissue type.

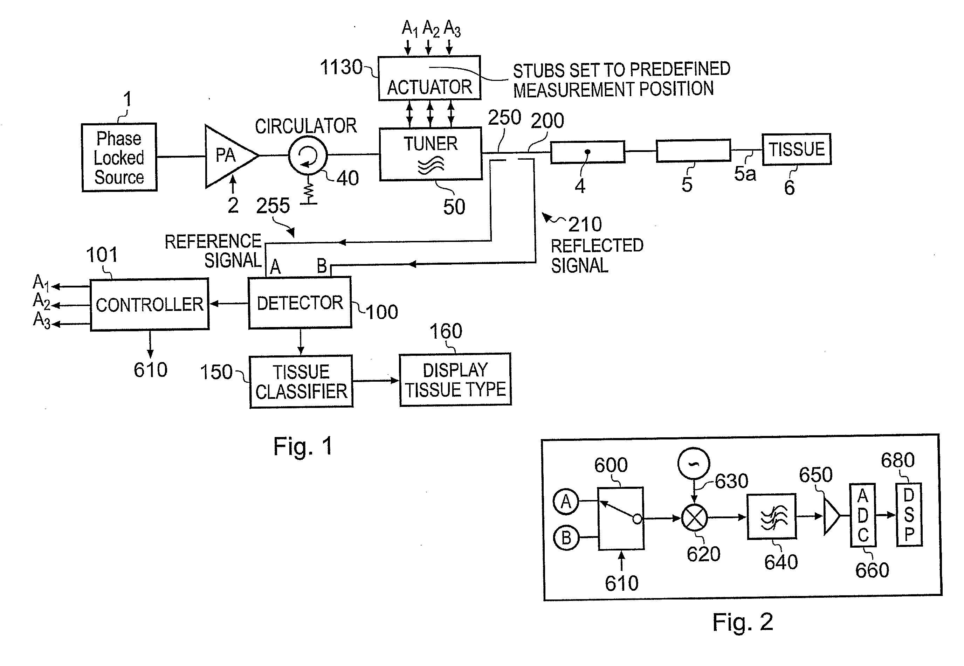

Fig. 2 shows a known configuration of the detector 100. A switch 600 is switchable to take either the signal from input A (the forward reference) or input B (the measurement data) of the detector 100. The switch 600 is controlled by signal 610 from controller 101 and can rapidly be switched between the two positions to get up to date information from each signal (i.e. the switch multiplexes the signals). Switch 600 outputs the reflected 210 or reference 255 signal to a mixer 620 where it is mixed with a signal 630 having a frequency different to the frequency of the reference 255 and reflected 210 signals. The frequency of the signal 630 is chosen such that it mixes with the reflected signal 210 and reference signal 255 to produce a lower frequency signal which can be output to a digital signal processor 680. Between the output of the mixer 620 and the digital signal processor 680 there is a low pass filter 640 for eliminating any high frequencies or other unwanted signals produced at the output of the mixer, that would otherwise be input into signal conditioning amplifier 650 and the analogue to digital converter 660.

The digital signal processor 680 calculates a complex impedance (having both real and imaginary components) on the basis of the input reflected and reference signals. The detector 100 outputs this information to the tissue classifier 150.

The tissue classifier 150 classifies the tissue 6 into one of a plurality of different tissue types or states the tissue may be in during the ablation process (e.g. skin, fat, muscle, cancerous tumour, cooked tissue etc) and is also able to detect when the probe is in air and not in contact with tissue on the basis of the complex impedance value calculated by the tissue classifier 150.

The tissue classifier 150 classifies the tissue by comparing the above mentioned complex impedance value (which is representative of the tissue 6 at the end of the probe) with a table of predetermined values assigning complex impedances or ranges thereof to specific tissue types. These predetermined values can be determined empirically or calculated theoretically on the basis of the known impedances of tissue types measured ex-vitro under controlled conditions. Chapter 6 of 'Physical Properties of Tissue'; a comprehensive reference book by France A Duck and published by Academic Press London in 1990 (ISBN 0-12-222800-6) provides data from which such theoretical values could be calculated.

The apparatus shown in Figs. 1 and 2 is capable of both ablating and measuring tissue 6 at the end of the probe 5. It uses the same radiation transmission path for both modes of operation, with the signals for measurement being coupled out of the main (ablation) line up.

## SUMMARY OF THE INVENTION

The inventors realised that there was a potential problem with coupling the measurement signal from the ablation line. Since the couplers remove only a portion of the signal on the ablation line, it is necessary to deliver power above a certain level even when the apparatus is operating in measurement mode to ensure that the coupled signal is detectable. It was identified that there is a risk that the delivered radiation might be powerful enough to damage the tissue being measured, i.e. the measurement signal may cause tissue ablation, e.g. it was discovered that power levels of around 1 W generated at the frequency of interest can produce tissue damage.

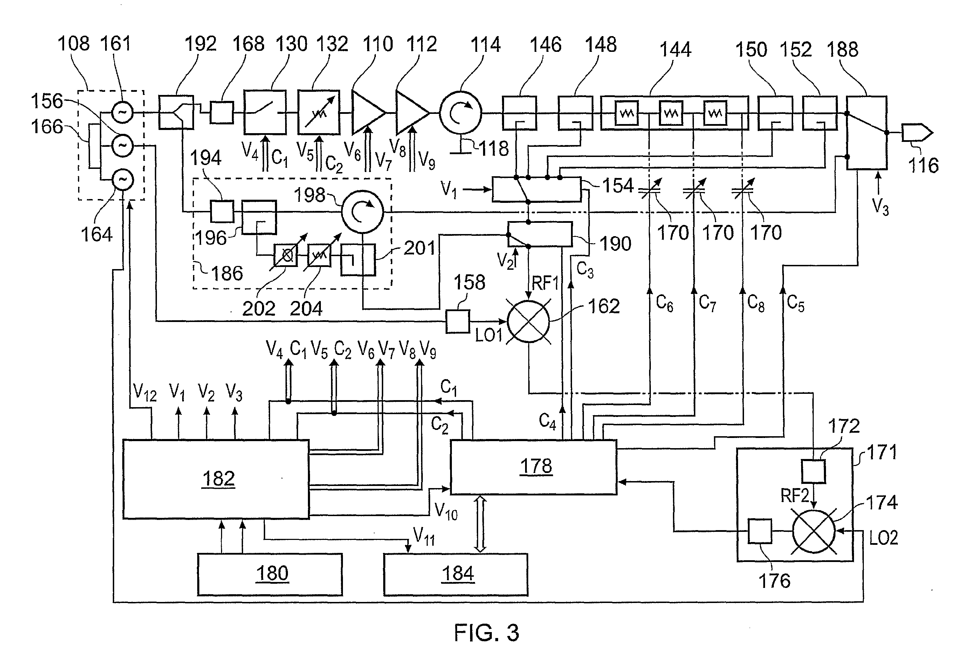

A solution to this problem was proposed by the present inventors in WO 2008/043999 A2 This document disclosed an electrosurgical apparatus capable of ablating and measuring biological tissue having two separate (independent) treatment channels between a microwave radiation source and a treatment probe: a first channel for radiation for ablation and a second channel for radiation for measurement (e.g. tissue classification). The power delivered by the second channel is much less (e.g. a factor of 100,000 less) than the power delivered by the first channel. In this arrangement, the reflected signal could be directly taken from the second channel. This was achieved using a circulator tuned or optimised to provide high signal isolation between the first and third ports at the frequency of interest and a carrier cancellation circuit arranged to isolate the reflected signal from the forward direction radiation. Apparatus according to this arrangement is shown in Fig. 3, which is described in detail below.

This solution improved measurement sensitivity and overcame the drawback associated with using relatively high levels of microwave power in the measurement circuit that may, for example, be high enough to cause tissue ablation. However, the inventors have discovered that drift occurs in the phase and magnitude of the delivered signal due to temperature variations and other changes in the microwave components or other components or devices in the apparatus. For example, device ageing or slight variations in the DC power supply can cause the characteristics of certain components to change, e.g. a variation in the DC power supply for the oscillator may cause an effect known as frequency pushing, which is a change in the output frequency of an oscillator as a function of the DC supply voltage. This drift occurs during a time period in which the apparatus would typically be used and can therefore lead to inaccuracies in the measured results. The present invention addresses this problem.

Expressed generally, the present invention proposes providing a forward reference signal to the detector for comparison with the reflected signal from the probe, wherein the reference signal is based on or derived from a signal delivered to the probe such that both the reflected signal and the reference signal will contain the same offset due to drift. By calculating and using the difference between the reference signal and the reflected signal for measurement, the offset due to drift is cancelled out.

According to the invention there is therefore provided a tissue classifying apparatus according to claim 1.

As the reference signal is derived from the same source as the reflected signal, they exhibit substantially the same magnitude/phase drift. This enables that drift to be compensated for in a measurement made by the detector. The reference signal may be obtained by providing a directional coupler on the first transmission path e.g. before the first port of the circulator to couple the reference signal from the first transmission path. In this case, the magnitude/phase drift can exactly cancel out if the time taken between making the reference signal measurement and the reflected signal measurement is relatively short, e.g. 1 ms, 10 ms or 100 ms, because the reference signal and reflected signal are connected to the same path (or route) through the microwave circuit, i.e. the reference signal is not passed through active devices that are not in the path of the reflected signal. Both the reference and reflected signal measurements may be made over a time frame of around 1 ms. If the reference signal is independently passed through active devices then it is expected that this signal will be affected by noise generated within the active device(s)and drift that may occur due to changes in junction temperature in, for example, a semiconductor amplifier.

The input to the detector may be regularly (e.g. periodically) switched between the reference signal and reflected signal. In one embodiment, the periods between switching the detector input between the reference signal and the reflected signal is relatively short, e.g. less than 1 second, e.g. between 0.1 ms and 100 ms. Signal drift within these periods should not have a significant effect on the validity of the measurement information presented to the user or used for further calculations or for controlling hardware components within the system.

The detector may be arranged to measure the difference in magnitude and phase between the reference signal and the reflected signal. This enables any drift error, which may be common to both signals, to be cancelled out. The measured difference is related to the reflection coefficient caused by the tissue impedance, and the tissue classifier is arranged to calculate the complex impedance of the tissue using the measured difference. It may be desirable to use these measurements to extract complex permittivity values.

The circulator may act to isolate the reference (forward) signal from the reflected signal. In other words, it can act to isolate (separate) the forward and reflected components of the main power line-up. The circulator, having a first port, a second port and a third port, the first transmission path including a pathway from the first port to the second port, and the second transmission path including a pathway from the second port to the third port. The third port may be terminated with a well-matched load to enable the circulator to act as an isolator. The circulator may be arranged, i.e. be designed, constructed, and tuned to prevent any signal from travelling between the first port and the third port. However, in practice a small amount of leakage may occur. The apparatus may therefore include a carrier cancellation circuit connected between the first port and third port of the circulator, the carrier cancellation circuit being arranged to cancel radiation from the source which leaks out of the third port of the circulator. The carrier cancellation circuit may comprise a first coupler arranged to couple forward directed radiation from the first transmission path, a signal adjustor arranged to modify the magnitude and/or phase of the coupled signal, and a second coupler arranged to couple the modified signal into the second transmission path, whereby the modified signal cancels radiation from the source which is leaking out of the third port of the circulator, i.e. the signal is antiphase and of the same magnitude. The signal adjustor may include a variable attenuator and/or a variable phase adjuster. The carrier cancellation circuit may also be used to cancel out any unwanted signal component caused by the cable assembly and the probe, i.e. the signal that is cancelled may be a composite of the signal that breaks through the circulator into the third port from the first port (the breakthrough signal) and the signal caused by the cable assembly and the probe (and any other components that exist along this path).

Alternatively or additionally, the reference signal can be used to mathematically remove any component of forward directed radiation present in the reflected signal. This is achieved by using digital signal processing techniques to subtract the component of the forward (reference) leakage signal from the desired reflected measurement signal. This may be achieved through measuring the quadrature I and Q values for the reflected signal with a fixed load impedance connected to the antenna or the end of the cable assembly (it is desirable for the load to be well matched with the characteristic impedance of the cable assembly, for example, a 50 $\Omega$ load such as that used for calibrating a laboratory vector network analyser. In this way, any signal measured will be due to signal breakthrough between the first and third ports of the circulator and any noise that may be generated by active components contained within the detector.

In one embodiment, the apparatus includes a mixer having a first input connected to receive the switched input for the detector, a second input connected to receive a mixing signal (e.g. from a local oscillator) for the mixer, and an output connected to the rest of the detector circuit, whereby a frequency of the periodically switched input for the detector is altered by the mixer before the input is received in the rest of the detector circuit. For example, the frequency of the microwave source may be too high to be processed by the analogue to digital converter (ADC) that may form a part of the detector. The mixing signal from the local oscillator may be a mixing down signal arranged to reduce the frequency of the switched input signal. The mixing down signal may be derived from the source of microwave radiation.

The apparatus is also configured to ablate biological tissue. The apparatus therefore includes a separate (independent) radiation delivery channel for conveying radiation to the probe from the source when the apparatus is operating in an ablation mode. The probe is selectively connectable to receive radiation from the source via either the first transmission path or a third transmission path that is independent of the first transmission path, the radiation receivable by the probe via the third transmission path being for ablating tissue. The apparatus can therefore work in an ablation mode where radiation is conveyed to the probe via the third transmission path or in a measurement mode where radiation is conveyed to the probe via the first transmission path.

The third transmission path may include an amplifier such that the radiation receivable via the third transmission path has higher amplitude than the radiation receivable via the first transmission path. The amplitude of radiation conveyed via the first transmission path may be many orders of magnitude smaller than that conveyed by the third transmission path. For example, power levels delivered at the distal end of the probe (the aerial) to enable tissue type/state measurements to be performed may be less than 10 mW, e.g. between 0.1 mW (-10 dBm) (or in some embodiments as little as 1 $\mu$W (-30 dBm)) and 10 mW (+10 dBm), whereas power levels delivered at the distal end of the probe to cause tissue ablation may range from 1 W (30 dBm) to 200 W (53 dBm) or more, e.g. 400 W (56 dBm).

The third transmission path may include an impedance adjuster having an adjustable complex impedance arranged to match the impedance of the apparatus to the impedance of the biological tissue.

The probe may be adapted to be insertable into tissue.

The range of microwave frequencies considered to be useful for the implementation of the current invention is between 500MHz and 60 GHz. Frequency ranges that may be particularly useful for implementing of the current invention are: 2.4-2.45 GHz, 5.725-5.875 GHz, 14-15 GHz, and 24-24.25 GHz. Spot frequencies that lie within these bands may be used for implementing the current invention, e.g. 2.45 GHz, 5.8 GHz, 14.5 GHz, and 24 GHz may be used. Single frequency offers advantage in terms of being able to set up high Q cavities and structures with relative ease, and by not having to design the microwave components to operate over wide bandwidths can have substantial effects on reducing component costs and overall system development costs in the future. The use of frequencies of around 915 MHz and 60 GHz may also be considered for future medical applications identified herein.

The benefits of the implementation of the enhanced configuration described in this work have now been demonstrated in a practical system. It has been recently demonstrated that the enhanced configuration described here allows for valid complex impedance measurements to be made even whilst the system is warming up, i.e. a useful measurement can be made as soon as the equipment has been switched on from a cold start. This feature may offer benefit over many existing test and measurement instruments, where it is often necessary to allow for the equipment to warm up, for example, for a period of ten minutes, before a valid measurement can be made. It is also worthwhile noting that previously it may have been desirable to repeat calibration several times over a period of a few hours when making sensitive measurements using laboratory test and measurement equipment. This invention may reduce or overcome this limiting requirement. Practical examples of such equipment may include; a vector network analyser, a power meter or an oscilloscope.

## BRIEF DESCRIPTION OF THE DRAWINGS

Fig. 1 shows a known electrosurgical apparatus for ablating or measuring biological tissue and is described above;
Fig. 2 shows a configuration of a detector that can be used in the apparatus of Fig. 1 and is also described above;
Fig. 3 shows an electrosurgical apparatus to which the present invention can be applied;
Fig. 4 shows an electrosurgical apparatus that is an embodiment of the invention; and
Fig. 5 shows graphs indicating phase and amplitude drift in an uncorrected apparatus (e.g. such as that shown in Fig. 3) and a corrected apparatus (e.g. such as that shown in Fig. 4).

## DETAILED DESCRIPTION; FURTHER OPTIONS AND PREFERENCES

Fig. 3 shows a diagram of an electrosurgical system that is suitable for using with the invention. It includes two treatment channels (an ablation channel and a measurement channel) which are described in detail below.

Both channels begin at a microwave source 108 and include treatment antenna (probe) 116. In the ablation channel, a primary frequency source 161 is used to generate a low power signal at a predetermined frequency, a driver amplifier 110 to amplify the output signal level produced by the primary frequency source 161, and a power amplifier 112 to amplify the signal produced by the driver amplifier 110 to a level that may cause controlled tissue destruction. The output from the power amplifier 112 is connected to a microwave circulator 114 which is used to protect the output transistors contained within power amplifier 112 from excessive amounts of reflected power caused by an impedance mismatch at the distal end of the treatment antenna 116 or due to an impedance mismatch caused by the position of the tuning elements (these may be tuning rods or screws) inside the tuning filter 144. The mismatch could also be caused by a discontinuity or change in impedance caused or introduced by any component connected between the output port 2 of the circulator and the distal end of the probe, i.e. the output connector, the cable assembly, etc. The circulator 114 only allows microwave power to flow in a clockwise direction, hence any reflected power coming back towards power amplifier 112 will be absorbed by power dump load 118 connected to the third port of said circulator 114. The power dump load must be well matched with the impedance of the third port of the circulator in order to prevent reflected power going back into the first port. In the instance where low output power levels are being generated, for example 1.5 W continuous wave, it may be possible to omit the microwave circulator and the 50 Ω power dump load from the design whilst maintaining the device operability (the worst case level of reflected power is such that damage may not be caused to the output stage of the power amplifier).

The ablation channel further includes a modulation switch 130 to enable the electrosurgical system to be operated in a pulsed mode. This mode of operation is particularly useful when the unit is operated at higher microwave power levels, for example, 15 W to 120 W, where thermal effects relating to the hand-piece and/or the probe shaft and/or the cable assembly should be considered. The ablation channel also has a power control attenuator 132, which is used to enable the user to control the level of power delivered into the tissue. Again, it may be desirable to include this feature where the unit is configured to be capable of delivering power levels up to, and possibly in excess of, 100 W. In practice, it may be possible to switch attenuator 132 on and off at a fast enough rate to enable modulation switch 130 to be omitted from the line-up. MEM technology may be used to implement the modulation switch 130 and power control attenuator 132. Source oscillator 108 may also take advantage of MEM technology to help miniaturise the overall size of the generator. Separate (or external) modulation switch and/or power control attenuator unit need not be required; these features (or operations) may be implemented by varying the level of voltage applied to the power generating devices. The variation of gain due to variation in DC or bias voltage may be around 15 dB. If wider variation is desired, a digital attenuator comprising of a bank of PIN diodes can be used. This may provide a variation of gain of up to (and in some cases in excess of) 64 dB.

The ablation channel further includes a dynamic impedance matching system to enable the microwave energy developed by the power amplifiers 110, 112 to be matched, in terms of impedance, with the load presented to the distal end of the treatment antenna 116 due to the current state of the biological tissue. The system may enable a conjugate match between the treatment instrument and tissue to be created. This configuration offers advantage in terms of efficient energy delivery into tissue, reduced treatment time, and the ability to accurately quantify energy dosage required to cause controlled tissue destruction due to the fact that the demanded power is the power that actually gets delivered into the tissue due to the fact that the matching algorithm enables the demanded power to be delivered into the tissue even when a mismatched condition occurs between the distal tip of the antenna and the tissue load.

The impedance matching system includes a tuning filter 144, four directional couplers 146, 148, 151, 152, a time multiplexing switch 154, and a double intermediate frequency (IF) heterodyne receiver with a first stage comprising a first local oscillator 156, a band-pass filter 158 used to remove any signal components of the primary frequency source 161, and a microwave frequency mixer 162. A third frequency source 164 provides a local oscillator signal for the second stage 171 of the double IF heterodyne receiver. Other components in the ablation channel include a reference frequency oscillator 166 to enable the three signal oscillators 156, 161, 164 to be synchronized together, and a second band-pass filter 168 connected between the output of the primary frequency source 161 and the input to modulation switch 130 to remove any signal components that may be present at the frequency of the first local oscillator signal 156.

In this arrangement, the time multiplexing switch 154 is used to enable signals from any one of the four coupled ports of forward power and reflected power signal couplers 146, 148, 151, 152 to be channelled into a double IF frequency down converter circuit (at mixer 162) to enable phase and magnitude extraction to be performed. It may be necessary to compare the information available at the later coupled ports 151, 152 or the earlier coupled ports 146, 148 to determine the adjustments required to the tuning elements 170 within (or outside) tuning filter 144 to enable the power source (i.e. power generated by amplifier 110 or a series connected chain of amplifiers 110, 112) to be impedance matched with the tissue load in order to ensure, for example, that the maximum amount of microwave power is transferred into the tissue load. As shown, adjustment may be implemented by adjusting the voltages on three diodes. The tuner 144 may also take the form of a plurality of tuning stubs contained within a tuning cavity, where an electromechanical actuator is used to move said tuning stubs up and down within the cavity, and a controller, for example a PID controller, is used to ensure that the movement of the tuning stubs (rods) is well defined. An inductive or capacitive reactance is introduced by the tuning stubs, and the value is dependent upon the length of stub that resides within the cavity. A number of topologies may be considered for the implementation of tuning filter 144, but to enable a compact system to be realized (for example, the overall size of the unit may end up being the size of a video cassette recorder), it may be preferable to use an arrangement of PIN or varactor diodes.

The operation of microwave frequency mixer 162 is to enable a portion of the high frequency microwave signal that is used to cause controlled tissue damage to be mixed down in frequency to a signal at a lower frequency, whilst preserving phase and magnitude information contained within the signal available from the coupled ports of the four directional couplers 146, 148, 151, 152. The desired output frequency from mixer 162 is the difference frequency between a first input RF1 from the couplers and a second input LO1 from the local oscillator 156. In the configuration given in Fig. 3, the difference between the RF1 input and the LO1 input is 50 MHz because the local oscillator 156 operates at 14.45 GHz while the primary frequency (to which the couplers are connected) is 14.5 GHz. The 50 MHz signal is used to extract phase and magnitude information. This invention is not limited to using the arrangement shown that uses four directional couplers 146, 148, 151, 152. For example, the latter two 151, 152 only may be used, or the former two 146, 148 only may be used. Also, a six port directional coupler, or any other suitable coupler arrangement comprising of a plurality of directional couplers, may be used in the implementation of the system.

The second stage 171 of the double IF heterodyne receiver comprises a third band-pass filter 172 used to remove signals other than the difference IF signal produced at the output of first mixer 162, second mixer 174 is used to mix the frequency down yet again to a value that can easily be dealt with using a standard analogue to digital converter. A fourth band-pass filter 176 is used to remove all signal components present at this point in the system at frequencies other than the difference IF signal produced at the output of second mixer 174. In this embodiment, the mixer produces a signal at a frequency that is the difference between a first input RF2 produced at the output of the first mixer 162 and a second input LO2 produced by a third frequency source 164. In this embodiment, the third frequency source operates at 40 MHz, so the difference is 10 MHz. The output from fourth band-pass filter 176 is fed into a digital processor 178, which may be a digital signal processor, a microprocessor, or a microcontroller, to enable the phase and magnitude information to be digitally extracted and converted into a format that can be used to control the variable elements 170 of the tuning filter 144 based on the information measured at the coupled ports of directional couplers 146, 148, 151, 152 (or a combination of) and directed to the heterodyne receiver using multiplexing switch 154. The analogue output from the receiver is digitised using a suitable analogue to digital converter (ADC) and the resultant digital signal is processed by the digital signal processing (DSP) unit or by the microprocessor (MP) unit. It may be worthwhile noting that the ADC may be contained within the DSP or MP units. It may only be required to use information available at the coupled ports of later directional couplers 151, 152 to control the variable tuning elements used to maintain the matched condition.

Power source 180 provides the required DC energy for the electrosurgical unit to operate. A voltage control unit 182 may comprise a plurality of DC to DC converters to enable a single voltage produced by power source 180 to be converted to a plurality of voltages necessary to operate the unit, for example the drain and gate-source voltages $V_6$-$V_9$ for the amplifiers 110, 112, the voltage $V_{10}$ to power up the microprocessor unit, etc. The voltage supplies and control signals are shown in detail in the Fig. 3.

The selection of the pole position of single-pole-four-throw (SP4T) time multiplexing switch 154, the open/close operation of modulation switch 130, and the level of attenuation introduced by variable attenuator 132 are determined by control signals $C_1$-$C_3$ generated by microprocessor 178.

The system includes a user interface 184 with which a user can operate the system. The user interface can include LED bar graphs, audible alarms single LEDs and micro-switches, voice (audible) recognition, voice (audible) feedback or an alphanumeric LCD display with micro-switches or membrane switches, a touch screen display, or any other suitable means of inputting information or data into the system and outputting or accessing information or data from the system.

The measurement channel provides a separate transmission path for conveying radiation from the primary frequency source 161 to the treatment antenna 116. The measurement channel bypasses the amplifiers and the dynamic tuning system associated with the ablation channel. In this configuration, a 3 dB splitter or coupler or power divider splits the output of the primary frequency source between the ablation channel and the measurement channel. A waveguide switch 188 and a co-axial switch 190 are used to enable switching between the two channels. The control signals $C_4$, $C_5$ to enable the switch position of waveguide switch 188 and co-axial switch 190 to be changed over are provided by the microprocessor (or digital signal processor) 178. This invention is not limited to using a waveguide switch and a co-axial switch to switch between the two modes of operation; for example, it may be possible to use two co-axial switches, two waveguide switches, a combination of PIN and waveguide switches, or a combination of PIN and co-axial switches. The measurement channel includes a low power transmitter 186 which is arranged to condition the signal supplied to and received from the antenna 116. An input signal from the primary frequency source 161 is fed into the input port of a band-pass filter 194 whose function is to pass energy produced at the measurement frequency, but reject energy produced at all other frequencies. The output from filter 194 is fed into the input of first directional coupler 196, which is configured as a forward power directional coupler and forms a part of a carrier cancellation circuit. The output from first directional coupler 196 is fed into the first port (the input port) of microwave circulator 198. The second port (the output port) of circulator 198 is connected to the measurement antenna via waveguide switch 188. The third port of microwave circulator 198 is connected to the input to second directional coupler 201, which is configured as a forward power directional coupler and forms a part of a carrier cancellation circuit. The output from second directional coupler 201 is fed into the RF input of first frequency mixer 162 (via co-axial switch 190) of the double IF heterodyne receiver.

The configuration and description of the double IF heterodyne receiver is explained above. In the measurement mode, the phase and magnitude information is extracted from the signal using digital signal processing and processed using microprocessor 178 to provide information relating to the tissue type and/or the state of the tissue that the distal tip of the antenna is making contact with. It may be noted that the digital signal processor may also process the signal or be used to perform a part of the processing function described above.

To enhance the isolation between the forward transmitted signal and the reflected signal in the measurement mode it is necessary to provide a high a level of isolation between the first and third ports of circulator 198. Preferably, the circulator 198 is tuned or optimized at the measurement frequency for low insertion loss in the signal path and high rejection in the isolated path. Additional isolation may be provided by means of a carrier cancellation circuit comprising first forward signal directional coupler 196, phase adjuster 202, adjustable attenuator 204, and second forward signal coupler 201. The carrier cancellation circuit works by taking a portion of the transmitted signal from the coupled port of signal coupler 196 and adjusting the phase and power level such that it is 180° out of phase and of the same amplitude as any unwanted signal that gets through to the third port of circulator 198 to enable the unwanted signal component to be cancelled out. The carrier cancellation signal is injected into (or at) the output of the third port of circulator 198 using second forward coupler 201. The carrier cancellation circuit may also be used to adjust for variations caused by the output antenna (co-axial shaft and probe tip)and the microwave cable assembly that connects the generator to the antenna. The carrier cancellation circuit may be set up when a representative cable assembly and probe is attached to the system.

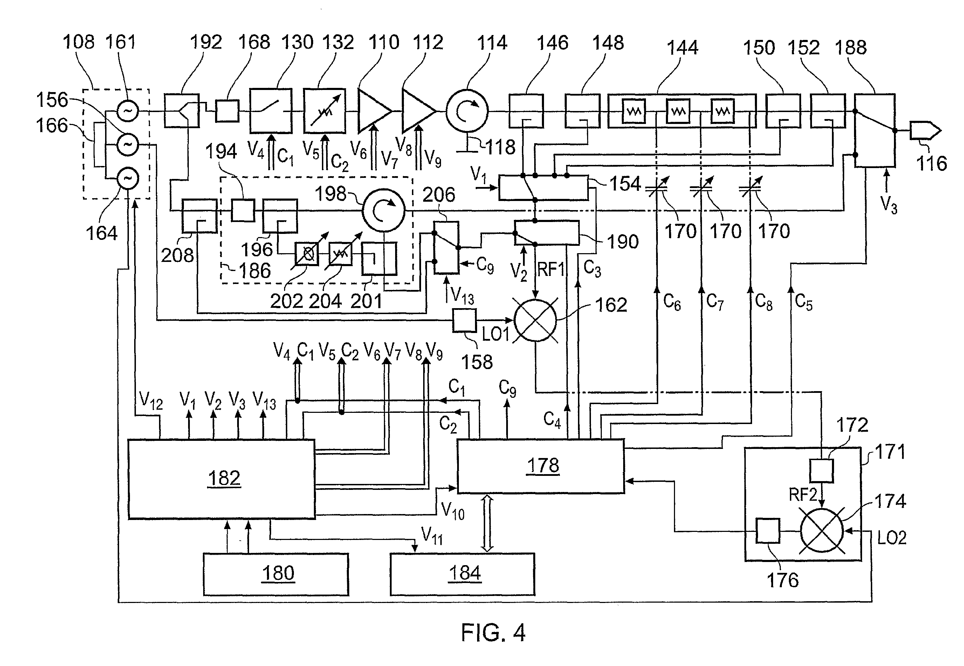

Fig. 4 shows an embodiment of the invention. It resembles the arrangement in Fig. 3 closely; the same reference numbers are used for common components and a description of those components is not repeated.

In the embodiment, the treatment channel is adapted to provide a reference signal (forward signal) derived from the primary frequency source 161 in addition to a reflected signal from the treatment antenna 116. Both signals are supplied via the double IF heterodyne receiver to the microprocessor (or digital signal processor) 178 where they are processed and used to determine the complex impedance of the tissue. This is achieved by measuring the difference between the signals at a location within the system where the two signals essentially contain the same offset in phase or amplitude due to drift, hence this variation can be cancelled out and only the desired forward and reflected power signals are measured. The complex impedance may then be calculated by extracting the phase and magnitude information from the two compensated signals. By dividing the magnitude of the signal produced from the reflected power measurement by the magnitude of the signal produced from the reference (forward) power measurement, and subtracting the phase of the forward power vector from the phase of the reflected power vector it is possible to establish the complex impedance with a high degree of accuracy.

The digital signal processor may detect quadrature I-Q signals from the input reference (forward) and reflected signals respectively. Transformations from the quadrature I-Q signals (Cartesian format) to magnitude and phase signals (polar format) and/or to real and imaginary values (complex impedance format) can then be performed in order to get the desired information out of the system.

For example, the digital signal processor may detect and normalize (based e.g. on factors determined by previous calibration using known load impedances) quadrature values $Q_f$ and $I_f$ for a reference (forward) detected voltage $V_f$ and quadrature values $Q_r$ and $I_r$ for a reflected detected voltage $V_r$. As an illustrative example, let the detected normalized values be:

$$Q_f = 0.6$$

$$I_f = 0.8$$

$$Q_r = -0.4$$

$$I_r = -0.3 \ .$$

These detected values can be converted to polar form $(R, \phi)$ using the equations

$$|R| = \sqrt{I^2 + Q^2}$$

and

$$\tan \phi = \frac{Q}{I} \ ,$$

such that

for $V_f$ the values are $R_f$ = 1.0 and $\phi_f$ = 36.87° and
for $V_r$ the values are $R_r$ = 0.5 and $\phi_r$ = 233.13°.

The required magnitude $R_t$ and phase $\phi_t$ information is given by the equations

$$R_t = \frac{R_r}{R_f}$$

and

$$\phi_t = \phi_r - \phi_f \ ,$$

which give $R_t$ = 0.5 and $\phi_t$ = 196.26° for the present example. These polar coordinates are then converted to complex impedance notation $(x_t \pm jy_t)$ to yield the required complex impedance information. Thus, in the present example

$$x_t = \frac{1 - R_t^2}{1 - (2R_t \cos \phi_t) + R_t^2} = 0.339 \ ,$$

and

$$jy_t = \frac{j2R_t \sin \phi_t}{1 - (2R_t \cos \phi_t) + R_t^2} = -j0.126 \ .$$

These values can be de-normalized to find the actual complex impedance of the measured tissue, For example, de-normalizing to 50 Ω gives $x_t + jy_t$ = 16.95-$j$6.3.

A switch 206 is arranged to provide a pathway for either the reflected signal or the reference (forward) signal to enter the mixer 162 of the first stage of the double IF heterodyne receiver. The switch 206 is a single pole two throw (SP2T) switch, e.g. part number S2K2 component from Advanced Control Components. The switch 206 switches between the reflected signal and reference (forward) signal periodically under the control of a control signal $C_9$ from the microprocessor 178 (or digital signal processor). The period to make the two measurements is short, i.e. less than 100 ms (e.g. within a time frame of 1 ms), hence there is not enough time for component drift to occur during the window of time over which the two measurements are taken.

The reflected signal is provided using the low power transmitter 186 discussed above in relation to Fig. 3.

A forward power coupler 208 is provided on the path from the primary frequency source 161 to the low power transmitter 186. The coupler 208 is configured to measure a portion (e.g. 10%) of the forward directed power generated by the primary frequency source 161. This measured portion is the reference (forward) signal. The reference signal therefore takes the same path as the signal which is eventually reflected from the tissue. This means if any offset exists it is present in both signals and can be cancelled by subtracting one signal from the other.

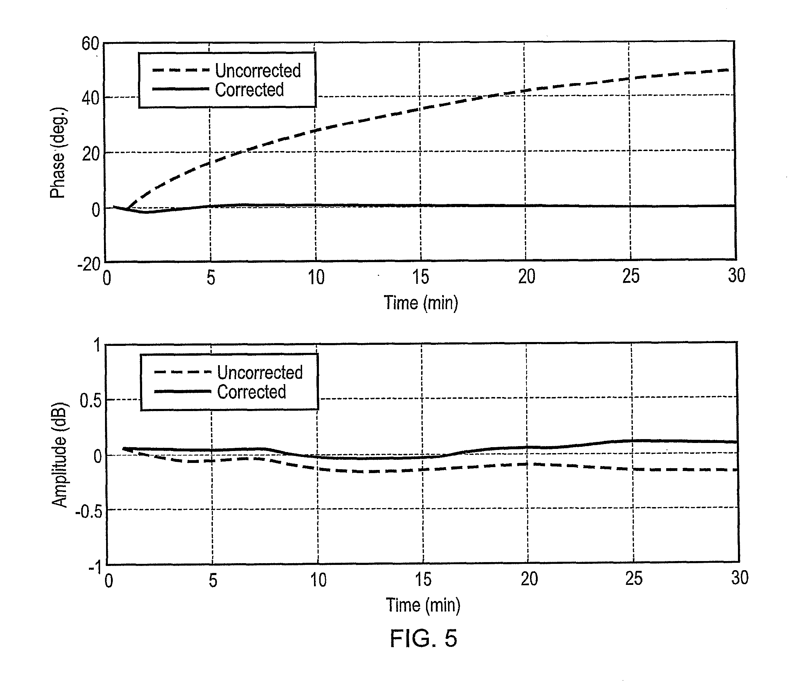

Fig. 5 shows the results of implementing the system enhancement described with reference to Fig. 4 into the system shown in Fig. 3. The top graph in Fig. 5 shows that the phase drift that can take place over a period of time can be removed by providing a reference signal for comparison with the reflected signal with the measurement of the reference signal being made at around the same time as the reflected signal (the signal of interest). The lower graph shows that a drift in amplitude observed over time in the previous system can be improved when the enhancement is incorporated. The system still exhibits minor drift due to variation in the characteristics of components within the detector, e.g. the channel select switch or one of the mixers. This minor drift does not appear to affect the measurements of complex impedance when the system operates in the tissue recognition mode to a level that adversely affects the measurement sensitivity of the system.

## Claims

1. Tissue classifying apparatus comprising:

   a source (108) of microwave radiation having a predetermined frequency;
   a probe (116) arranged to deliver radiation from the source (108) in a forward direction into tissue and to receive reflected radiation from the tissue;
   a detector (178) arranged to receive an input that is switchable between a reference signal that is derived from the forward directed radiation from the source (108) and reflected radiation received from the probe (116) along a reflection transmission path, the detector (178) being arranged to detect the magnitude and phase of both the reflected radiation and the reference signal;
   a circulator (198); and
   a tissue classifier arranged to classify the tissue on the basis of the magnitude and phase of the signals detected by the detector (178),
   **characterised in that**:

   the probe is selectively connectable to receive radiation from the source (108) along either a measurement transmission path or a treatment transmission path, the measurement transmission path being independent of the treatment transmission path, and the circulator (198) is located between the source (108), the probe (116) and the detector (178) to isolate forward directed radiation along the measurement transmission path from reflected radiation along the reflection transmission path to prevent the forward directed radiation from travelling along the reflection transmission path.

2. Apparatus according to claim 1, wherein the circulator (198) has a first port, a second port and a third port, the measurement transmission path including a pathway from the first port to the second port, and the reflection transmission path including a pathway from the second port to the third port.

3. Apparatus according to claim 2 including a carrier cancellation circuit connected between the first port and third port of the circulator, the carrier cancellation circuit being arranged to cancel radiation from the source which leaks from the first port into the third port of the circulator.

4. Apparatus according to claim 3, wherein the carrier cancellation circuit comprises a first coupler (196) arranged to couple forward directed radiation from the measurement transmission path, a signal adjustor (202, 204) arranged to modify the magnitude and/or phase of the coupled signal, and a second coupler (201) arranged to couple the modified signal into the reflection transmission path, whereby the modified signal cancels radiation from the source which is leaking out of the third port of the circulator.

5. Apparatus according to claim 3 or 4, wherein the carrier cancellation circuit is arranged to cancel a component in the reflected radiation caused by the cable assembly and/or the probe.

6. Apparatus according to any preceding claim, wherein the reference signal is coupled from the measurement transmission path.

7. Apparatus according to any preceding claim including a directional coupler (208) on the measurement transmission path, wherein the signal sampled by the directional coupler (208) is the reference signal, which is provided to the detector (178) to be subtracted from the reflected radiation.

8. Apparatus according to any preceding claim including a mixer (162) having a first input connected to receive the periodically switched input for the detector (178), a second input connected to receive a mixing down signal for the mixer, and an output connected to the detector (178), whereby a frequency of the periodically switched input for the detector is altered by the mixer before the input is received in the detector.

9. Apparatus according to claim 8, wherein the mixing down signal is derived from the source (108) of microwave radiation.

10. Apparatus according to any preceding claim, wherein the treatment transmission path includes an amplifier (110, 112) arranged to cause the amplitude of radiation receivable via the treatment transmission path to be higher than the radiation receivable via the measurement transmission path.

11. Apparatus according to claim 10, wherein the treatment transmission path includes an impedance adjuster (144) having an adjustable complex impedance arranged to match the impedance of the apparatus to the tissue.

12. Apparatus according to any preceding claim, wherein the probe (116) is insertable into tissue.

13. Apparatus according to any preceding claim, wherein the source (108) of microwave radiation is phase locked to a single frequency.

14. Apparatus according to any preceding claim, wherein the amplitude of microwave power launched into tissue by the radiation is less than 100 mW (20 dBm) to prevent thermal injury or damage to healthy tissue structures.

**Patentansprüche**

1. Gewebeklassifizierungsgerät, umfassend:

   eine Mikrowellenstrahlungsquelle (108) mit einer vorbestimmten Frequenz;
   eine Sonde (116), die so angeordnet ist, dass sie Strahlung von der Quelle (108) in eine Vorwärtsrichtung in Gewebe leitet und reflektierte Strahlung aus dem Gewebe empfängt;
   einen Detektor (178), der so angeordnet ist, um ein Eingangssignal zu empfangen, das zwischen einem Referenzsignal, das von der vorwärts gerichteten Strahlung aus der Quelle (108) abgeleitet ist, und der reflektierten Strahlung schaltbar ist, die von der Sonde (116) entlang eines Reflexionsübertragungspfades empfangen wird, wobei der Detektor (178) so angeordnet ist, dass er die Größe und Phase sowohl der reflektierten Strahlung als auch des Referenzsignals detektiert;

   einen Zirkulator (198); und
   einen Gewebeklassifikator, der so angeordnet ist, dass er das Gewebe auf Grundlage der Größe und Phase der vom Detektor (178) detektierten Signale klassifiziert,
   **dadurch gekennzeichnet, dass**:

   die Sonde selektiv verbindbar ist, um Strahlung von der Quelle (108) entweder über einen Messübertragungspfad oder einen Behandlungsübertragungspfad empfangen kann, wobei der Messübertragungspfad unabhängig vom Behandlungsübertragungspfad ist, und
   der Zirkulator (198) zwischen der Quelle (108), der Sonde (116) und dem Detektor (178) angeordnet ist, um vorwärts gerichtete Strahlung über den Messübertragungspfad von reflektierter Strahlung über den Reflexionsübertragungspfad isoliert, um zu verhindern, dass sich die vorwärts gerichtete Strahlung über den Reflexionsübertragungspfad ausbreitet.

2. Gerät nach Anspruch 1, worin der Zirkulator (198) einen ersten Anschluss, einen zweiten Anschluss und einen dritten Anschluss aufweist, wobei der Messübertragungspfad eine Leitung vom ersten Anschluss zum zweiten Anschluss umfasst und der Reflexionsübertragungspfad eine Leitung vom zweiten Anschluss zum dritten Anschluss umfasst.

3. Gerät nach Anspruch 2, welches einen Trägerauslöschungsschaltkreis umfasst, der zwischen dem ersten Anschluss und dem dritten Anschluss des Zirkulators verbunden ist, wobei der Trägerauslöschungsschaltkreis so angeordnet ist, um Strahlung von der Quelle auszulöschen, die vom ersten Anschluss in den dritten Anschluss des Zirkulators leckt.

4. Gerät nach Anspruch 3, worin der Trägerauslöschungsschaltkreis einen ersten Koppler (196), der so angeordnet ist, dass er vorwärts gerichtete Strahlung vom Messübertragungspfad koppelt, eine Signaleinstelleinheit (202, 204), die so angeordnet ist, dass sie die Größe und/oder Phase des gekoppelten Signals modifiziert, sowie einen zweiten Koppler (201) umfasst, der so angeordnet ist, dass er das modifizierte Signal in den Reflexionsübertragungspfad koppelt, wobei das modifizierte Signal Strahlung von der Quelle auslöscht, die aus dem dritten Anschluss des Zirkulators leckt.

5. Gerät nach Anspruch 3 oder 4, worin der Trägerauslöschungsschaltkreis so angeordnet ist, dass er eine Komponente der reflektierten Strahlung auslöscht, die durch die Kabelanordnung und/oder die Sonde verursacht wird.

**6.** Gerät nach einem der vorangehenden Ansprüche, worin das Referenzsignal vom Messübertragungspfad gekoppelt ist.

**7.** Gerät nach einem der vorangehenden Ansprüche, welches einen Richtungskoppler (208) auf dem Messübertragungspfad umfasst, worin das vom Richtungskoppler (208) abgetastete Signal das Referenzsignal ist, welches dem Detektor (178) bereitgestellt wird, damit es von der reflektierten Strahlung subtrahiert werden kann.

**8.** Gerät nach einem der vorangehenden Ansprüche, welches einen Mischer (162) mit einem ersten Eingang, der zum Empfangen des periodisch geschalteten Eingangssignals für den Detektor (178) verbunden ist, einen zweiten Eingang, der zum Empfangen eines herunterzumischenden Signals für den Mischer verbunden ist, und einen Ausgang umfasst, der mit dem Detektor (178) verbunden ist, wobei eine Frequenz des periodisch geschalteten Eingangssignal für den Detektor vom Mischer verändert wird, bevor das Eingangssignal vom Detektor empfangen wird.

**9.** Gerät nach Anspruch 8, worin das herunterzumischende Signal von der Mikrowellenstrahlungsquelle (108) abgeleitet ist.

**10.** Gerät nach einem der vorangehenden Ansprüche, worin der Behandlungsübertragungspfad einen Verstärker (110, 112) umfasst, der so angeordnet ist, dass er bewirkt, dass die Amplitude der über den Behandlungsübertragungspfad empfänglichen Strahlung höher als die der über den Messübertragungspfad empfänglichen Strahlung ist.

**11.** Gerät nach Anspruch 10, worin der Behandlungsübertragungspfad eine Impedanzeinstelleinheit (144) umfasst, die eine einstellbare komplexe Impedanz aufweist und so beschaffen ist, dass sie die Impedanz des Geräts an das Gewebe anpasst.

**12.** Gerät nach einem der vorangehenden Ansprüche, worin die Sonde (116) in Gewebe einführbar ist.

**13.** Gerät nach einem der vorangehenden Ansprüche, worin die Mikrowellenstrahlungsquelle (108) phasenstarr an eine einzelne Frequenz gekoppelt ist.

**14.** Gerät nach einem der vorangehenden Ansprüche, worin die Amplitude der Mikrowellenleistung, die durch die Strahlung in das Gewebe eingebracht wird, geringer als 100mW (20 dBm) ist, um thermische Verletzungen oder Schäden an gesunden Gewebestrukturen zu verhindern.

**Revendications**

**1.** Appareil de classification de tissu comprenant :

une source (108) de rayonnement hyperfréquence ayant une fréquence prédéterminée ; une sonde (116) agencée pour délivrer un rayonnement provenant de la source (108) dans une direction vers l'avant dans un tissu et pour recevoir un rayonnement réfléchi à partir du tissu ; un détecteur (178) agencé pour recevoir une entrée qui peut être commutée entre un signal de référence qui est déduit du rayonnement dirigé vers l'avant provenant de la source (108) et un rayonnement réfléchi reçu de la sonde (116) le long d'un trajet de transmission de réflexion, le détecteur (178) étant agencé pour détecter l'amplitude et la phase à la fois du rayonnement réfléchi et du signal de référence ; un circulateur (198) ; et un classifieur de tissu agencé pour classifier le tissu sur la base de l'amplitude et de la phase des signaux détectés par le détecteur (178), **caractérisé en ce que** :

la sonde peut être connectée de manière sélective pour recevoir un rayonnement de la source (108) le long de l'un ou l'autre d'un trajet de transmission de mesure et d'un trajet de transmission de traitement, le trajet de transmission de mesure étant indépendant du trajet de transmission de traitement, et le circulateur (198) est situé entre la source (108), la sonde (116) et le détecteur (178) pour isoler un rayonnement dirigé vers l'avant le long du trajet de transmission de mesure d'un rayonnement réfléchi le long du trajet de transmission de réflexion pour éviter que le rayonnement dirigé vers l'avant ne se propage le long du trajet de transmission de réflexion.

**2.** Appareil selon la revendication 1, dans lequel le circulateur (198) comporte un premier port, un deuxième port et un troisième port, le trajet de transmission de mesure comprenant un trajet du premier port au deuxième port, et le trajet de transmission de réflexion comprenant un trajet du deuxième port au troisième port.

**3.** Appareil selon la revendication 2 comprenant un circuit d'annulation de porteuse connecté entre le premier port et le troisième port du circulateur, le circuit d'annulation de porteuse étant agencé pour annuler un rayonnement provenant de la source qui fuit du premier port dans le troisième port du circulateur.

**4.** Appareil selon la revendication 3, dans lequel le circuit d'annulation de porteuse comprend un premier coupleur (196) agencé pour coupler un rayonnement dirigé vers l'avant provenant du trajet de transmission de mesure, un dispositif d'ajustement de signal (202, 204) agencé pour modifier l'amplitude et/ou la phase du signal couplé, et un deuxième coupleur (201) agencé pour coupler le signal modifié dans le trajet de transmission de réflexion, moyennant quoi le signal modifié annule un rayonnement provenant de la source qui fuit hors du troisième port du circulateur.

**5.** Appareil selon la revendication 3 ou 4, dans lequel le circuit d'annulation de porteuse est agencé pour annuler une composante dans le rayonnement réfléchi provoquée par l'ensemble des câbles et/ou la sonde.

**6.** Appareil selon l'une quelconque des revendications précédentes, dans lequel le signal de référence est couplé à partir du trajet de transmission de mesure.

**7.** Appareil selon l'une quelconque des revendications précédentes comprenant un coupleur directionnel (208) sur le trajet de transmission de mesure, dans lequel le signal échantillonné par le coupleur directionnel (208) est le signal de référence, qui est fourni au détecteur (178) pour être soustrait du rayonnement réfléchi.

**8.** Appareil selon l'une quelconque des revendications précédentes comprenant un mélangeur (162) ayant une première entrée connectée pour recevoir l'entrée commutée périodiquement pour le détecteur (178), une deuxième entrée connectée pour recevoir un signal de mélange pour le mélangeur, et une sortie connectée au détecteur (178), moyennant quoi une fréquence de l'entrée commutée périodiquement pour le détecteur est modifiée par le mélangeur avant que l'entrée soit reçue dans le détecteur.

**9.** Appareil selon la revendication 8, dans lequel le signal de mélange est déduit de la source (108) de rayonnement hyperfréquence.

**10.** Appareil selon l'une quelconque des revendications précédentes, dans lequel le trajet de transmission de traitement comprend un amplificateur (110, 112) agencé pour amener l'amplitude du rayonnement pouvant être reçu par l'intermédiaire du trajet de transmission de traitement à être supérieure à celle du rayonnement pouvant être reçu par l'intermédiaire du trajet de transmission de mesure.

**11.** Appareil selon la revendication 10, dans lequel le trajet de transmission de traitement comprend un dispositif d'ajustement d'impédance (144) ayant une impédance complexe ajustable agencée pour adapter l'impédance de l'appareil au tissu.

**12.** Appareil selon l'une quelconque des revendications précédentes, dans lequel la sonde (116) peut être insérée dans un tissu.

**13.** Appareil selon l'une quelconque des revendications précédentes, dans lequel la source (108) de rayonnement hyperfréquence est verrouillée en phase avec une fréquence unique.

**14.** Appareil selon l'une quelconque des revendications précédentes, dans lequel l'amplitude de la puissance hyperfréquence lancée dans le tissu par le rayonnement est inférieure à 100 mW (20 dBm) pour éviter une blessure ou un endommagement thermique des structures de tissus saines.

Fig. 1

Fig. 2

EP 2 117 456 B1

FIG. 3

FIG. 4

FIG. 5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2004047659 A
- WO 2005115235 A
- WO 2008043999 A2

**Non-patent literature cited in the description**

- **FRANCE A DUCK.** Physical Properties of Tissue. Academic Press, 1990